# EUROPEAN PATENT SPECIFICATION

(11) **EP 2 734 619 B1**
(45) Date of publication and mention of the grant of the patent: **06.09.2017**
(21) Application number: 12734892.8
(22) Date of filing: 04.07.2012
(51) Int. Cl.: C12N 7/02

(54) **PROCESS FOR PRODUCING ORTHOMYXOVIRAL ANTIGEN AND VACCINES**
VERFAHREN ZUR HERSTELLUNG VON ORTHOMYXOVIRALEM ANTIGEN UND IMPFSTOFFEN
PROCÉDÉ DE PRODUCTION D'ANTIGÈNE ORTHOMYXOVIRAL ET DE VACCINS

(30) Priority: 20.07.2011 EP 11174660; 20.07.2011 US 201161509630 P
(43) Date of publication of application: 28.05.2014
(73) Proprietor: BGP Products B.V., 2132 JD Hoofddorp (NL)
(72) Inventor: CUI, Yi-Qing, NL-1381 CP Weesp (NL); WANG, Yiding, NL-1381 CP Weesp (NL); THUS, Jo, NL-1381 CP Weesp (NL); VAN 'T HOF, Ron, 3741 AD Kamerik (NL); SHAKER, Sabah, NL-1381 CP Weesp (NL); VAN ROOIJ, Geertje Jacoba, NL-1381 CP Weesp (NL)
(74) Representative: Prüfer & Partner mbB Patentanwälte · Rechtsanwälte
(86) International application number: PCT/EP2012/063045
(87) International publication number: WO 2013/010797

(56) References cited:
- WO-A1-2011/051235
- WO-A2-2009/115917
- US-A- 4 140 762
- 'Ion exchange chromatography Screening and optimization of the loading conditions on Capto S', [Online] 01 January 2006, XP055163367 Retrieved from the Internet: <URL:https://www.gelifesciences.com/gehcls_ images/GELS/Related Content/Files/1314750913712/litdoc28407816A A_20110831033108.pdf> [retrieved on 2015-01-20]

## Description

### Field of the invention

The present invention belongs to the field of pharmaceutical industry and relates to a method for the production of a pharmaceutical composition comprising viral antigen derived from enveloped virus.

### Description of the background art

Currently, viruses that are used for the production of pharmaceutical compositions such as vaccine preparations or intermediates thereof are propagated in egg-based or cell-based culture systems. independent of the respective system used, the processing of viral antigens from each of the culture systems is a very important and critical step in the production of pharmaceutical compositions such as vaccine preparations. As to the egg-based system, the major requirement of the processing is the sufficient removal of the embryonated-egg proteins such as ovalbumin, and as to the cell-based system, the processing has to fulfill requirements such as significant reduction of host-cell DNA and host-cell proteins. Furthermore, non-product viruses have to be significantly cleared.

With respect to the purification of viral proteins, EP 1 982 727 A1 discloses a purification process of viral membrane protein comprising the steps of separating cells, microcarriers and cell agglomerates from the culture medium by sedimentation, subjecting said sediment to chemical inactivation and solubilization of said sediment, adding sodium cholate, centrifugating of the mixture, reducing the amount of detergent by Amberlite treatment and carrying out an affinity chromatography, following by eluting the target protein.

US 2011/0014230 A1 describes a process for preparing vaccine antigens, essentially comprising the step of inactivating purified virions, followed by splitting said virions with detergents.

US 2009/0060950 A1 discloses a method for producing viral vaccines comprising the steps of propagating a virus in a cell, collecting said virus in the cell supernatant, inactivating said virus, purifying it by density gradient ultracentrifugation and treating it with a detergent.

US 4,327,182 discloses a method for preparing a purified influenza subunit vaccine using membrane filtration techniques.

WO 2009/115917 and US 4,140,762 both discloses a method for preparing vaccine antigens, wherein additional steps take place between a concentration step and a solubilization step.

US 6,048,537 refers to a method for preparing purified influenza antigens, comprising the steps of concentration, purification, and fragmentation.

Further documents that refer to purification methods are US 2010/0119552 A1; US 2010/0158944 A1; Kalbfuss et al., 2007, Biotech&Bioeng. Vol. 96, No. 5, pp 932-944; Onions et al., 2010, Biologicals, Vol.38, pp 544-551; US 2008/0118970; WO 03/097797; Goerke et al., 2005, Biotechnology and Bioengeneering, Vol. 91, No. 1, pp 12-21; Krober et al., Chem. Eng. Technology 2010, Vol. 33, pp 941-959; and WO 2010/052214 A2. "Ion exchange chromatography Screening and optimization of the loading conditions on Capto S", 1 January 2006, describes a medium that can be used in chromatography.

WO 2011/051235 discloses a process for producing split or subunit influenza virus preparation comprising the steps of providing a whole virus preparation, splitting said virus preparation, and filtering said preparation.
Despite the above described methods, there is still a need and thus an object for an improved method for the production of a viral antigen, notably a viral antigen preparation and in particular a pharmaceutical composition such as a vaccine. In particular, there is a need for a method of production which gives high product recovery at relatively low production cost, which is viral strain independent, and requires a minimum number of steps only, while fulfilling the stringent requirements of low levels of host-cell DNA and proteins and extraneous agents.

### Summary of the invention

The present invention provides the following aspects, subject-matters and preferred embodiments, which respectively taken alone or in combination, contribute to solving the object of the present invention:
(1) A method for the production of haemagglutinin and/or neuraminidase derived from orthomyxovirus, comprising the following steps, in the indicated order:
   a) providing a fluid derived from a cell-based orthomyxovirus propagation culture or an egg-based orthomyxovirus propagation culture comprising said virus,
   b) clarifying the fluid comprising orthomyxovirus by applying centrifugation, tangential flow filtration, or depth filtration, resulting in a liquid harvest comprising said virus,
   c) reducing the volume of the liquid harvest obtained after step b) by a factor of at least 4 to obtain a concentrated liquid harvest, wherein this volume reducing step is carried out in one single step,
   d) solubilizing the orthomyxovirus being present in the liquid harvest by adding a detergent or mixture of detergents comprising a cationic detergent to the concentrated liquid harvest obtained in step c), wherein the final concentration of said cationic detergent is at least 2000 µg/ml,
   e) separating viral cores from the composition obtained after step d),
   f) optionally removing cationic detergent from the composition obtained after step e),
   g) purifying haemagglutinin and/or neuraminidase by applying chromatography,
   wherein step c) is followed directly by step d),
   and
   wherein no purification step that is directed at specifically purifying orthomyxovirus is carried out prior to step d).

In a preferred embodiment, no main purification step, in particular no main purification step that is directed at purifying enveloped virus, preferably no chromatography, is carried out prior to step e). After step e), a main purification step takes place. This main purification step, taking place after step e), is directed at purifying viral antigen. It is not directed at purifying whole virus or whole virions, respectively. With regard to the definition of the term "main purification step" (or "main purification method", respectively), reference is made to the definition elsewhere herein.

In a further preferred embodiment, the only chromatography step that is carried out is carried out in step g).

In a further preferred embodiment, no density gradient centrifugation (such as a density gradient ultracentrifugation, e.g. a sucrose gradient ultracentrifugation) is carried out prior to step e).

In a further embodiment, an additional viral inactivation step, such as a chemical inactivation step, and/or an additional viral clearance step may be applied. However, it might well be that by applying the present invention, the use of such an additional viral inactivation step and/or additional viral clearance step is not necessary in order to arrive at a sufficient inactivation of any pathogens and sufficient clearance, i.e. in order to arrive at an inactivation and clearance that fulfils respective regulations. This is an advantage as omitting such an additional viral inactivation and/or additional viral clearance step further contributes to an improved process, e.g. with regard to minimizing the stress exposed on desired viral antigens, and/or shortening the process. If however an additional viral inactivation step, such as a chemical inactivation, and/or an additional viral clearance step is carried out, this step/s does/do not take place between steps c) and d): As disclosed elsewhere, the direct following of step c) by step d) amongst others contributes to an enhanced recovery of viral antigen. In another aspect, said additional inactivation and/or additional clearance step is not carried out prior to step e). In a further embodiment, said additional inactivation and/or additional clearance step is for instance carried out after step g). Further, if such an additional viral inactivation step and/or additional viral clearance step is carried out, the concentration of chemicals needed and used in order to arrive at a final product that fulfils regulations is lower compared to the concentration of chemicals conventionally needed and used in order to arrive at a final product that fulfils regulations. This can be advantageous e.g. with respect to reducing the exposed stress and the chemical modifications on viral antigens. It can further be beneficial for product efficacy and probably stability.

Within the meaning of the present invention, the term "purifying viral antigen" denotes main purification methods or main purification steps, such as chromatography, or density gradient ultracentrifugation purification methods. In one embodiment, the main purification method does not comprise selective membrane filtration. The chromatography used can be ion exchange chromatography, hydrophobic interaction chromatography, mixed mode chromatography, pseudo affinity chromatography, affinity chromatography, liquid-liquid chromatography and the like. Within the meaning of the present invention, the term "main purification method" or "main purification step" refers to methods that are the major purification methods that are used in order to purify the (finally) desired product. In other words, main purifications methods/steps are directed at specifically purifying the (finally) desired product. In the present invention, for instance, said (finally) desired product is the viral antigen that is to be purified, but not the whole enveloped virus, prior to solubilization. Other methods which are not the "main purification method" may fulfill a different purpose, such as a clarification purpose, a concentration purpose or a pelleting purpose, with the sometimes accompanying effect of additionally purifying the desired (final) product. The result of such a method step not being the "main purification step" or "main purification method" is that the purification effect of the desired (final) product after having carried out such a method/step is lower compared to the purification effect that is reached when having carried out the "main purification step/method". After having carried out the main purification step, preferable no further purification steps are carried out (for instance in order to fulfill regulations as to impurities still being present in the composition). Clearance and inactivation steps such as chemical inactivation steps are not regarded as main purification steps with this respect. According to the present invention, such "main purification steps/methods" are not carried out prior to step e).
(2) The method according to item (1), wherein after step d) and prior to step g), detergent that has been added in step d) is removed.
   In one embodiment, no chromatography step is used for removing the detergent.
(3) Method according to item (1) or (2), wherein the volume in step c) is reduced by a factor of at least 5.
   In a preferred embodiment, the volume is reduced by a factor of at least 9, more preferably at least 12, even more preferably at least 13, or at least 15, and most preferably at least 20, or at least 25. A typical maximum volume reduction is 30, 35, 40 or 45.
   The reduction of the volume in step c) is carried out in one single step. Within the meaning of the present invention, the expression "one single step" denotes that there is only one volume reduction step that was carried out, and that this single volume-reduction step is not repeated.
(4) Method according to any of items (1) to (3), wherein the final concentration of the cationic detergent is at least about 2500 µg/ml, also preferred at least about 3000 µg/ml, also preferred at least about 3500 µg/ml, also preferred at least about 4000 µg/ml, also preferred at least about 4500 µg/ml, also preferred at least about 5000 µg/ml, also preferred at least about 5500 µg/ml, and also preferred at least about 6000 µg/ml.
(5) Method according to any of the preceding items, wherein the cationic detergent is based on quaternary ammonium cations like cetyltrimethylammonium bromide or other alkyl trimethylammonium salts, alkyl amine salts like stearyl amine acetate or coconut alkyl amine acetate, benzalkonium chlorides and bromides, for example benzethonium chloride or methylbenzethonium chloride, stearylaminepolyglycolether or oleylaminepolyglycolether, preferably the cationic detergent is cetyl trimethylammonium bromide (CTAB).
   In a further embodiment, the detergent is a mixture of detergents, wherein said mixture comprises, as detergents, a cationic detergent as described herein, preferably CTAB, and a non-ionic detergent as described herein, preferably a member of the Tween-family, preferably Tween-80.
(6) Method according to any of the preceding items, wherein step b) is directly followed by step c), which is directly followed by step d).
   In a further embodiment, step d) is directly followed by step e).
(7) Method according to any of the preceding items, wherein no detergent exchange takes place.
(8) Method according to any of the preceding items, wherein no nuclease or other DNA digesting enzyme, preferably no Benzonase®, is added prior to step d), preferably no nuclease is added at any stage of the process.
(9) Method according to any of the preceding items, wherein the orthomyxovirus is an influenza virus such as influenza virus A, B or C.
(10) Method according to any of the preceding items, wherein the cell-based orthomyxovirus propagation culture comprises cells of a mammalian cell line, preferably of an animal cell line.
(11) Method according to item (10), wherein the cells of the mammalian cell line are selected from the group consisting of Vero, PerC6, BHK, 293, COS, PCK, MRC-5, MDCK, MDBK and WI-38, preferably the cells are MDCK cells.
(12) Method according to item (10) or (11), wherein the cells are cultivated as adherent cells.
(13) Method according to any of the preceding items, wherein the egg-based orthomyxovirus propagation culture comprises hen eggs.
(14) The method according to any of the preceding items, wherein the fluid in step a) is produced by using a disposable bioreactor.
   The use of a disposable bioreactor can further contribute to the improvement of the method according to the present invention compared to conventional methods, for instance with regard to the obtained product itself, e.g. in terms of quality, and/or purity. This is in particular beneficial if the method is carried out in large-scale, e.g. in industrial scale.
(15) Method according to any of the preceding items, wherein in one embodiment, the conditions of the centrifugation of step b) are e.g. equal to or less than 10000 g of RCF (relative centrifugal force) for less than 2 min, preferably equal to or less than 3000 g of RCF for equal to or less than 2 min, more preferably equal to or less than 500 g of RCF for equal to or less than 2 min, even more preferably equal to or less than 300 g of RCF for equal to or less than 2 min. Within the meaning of the present invention, "g" refers to the standard acceleration due to gravity at the earth surface;
   in one embodiment, for the tangential flow filtration of step b), the pore size of the microfiltration membranes is e.g. at least 0.2 µm, preferably at least 0.45 µm, and even more preferably at least 0.65 µm;
   in one embodiment, for the depth filtration of step b), e.g. the 0.2 µm-7 µm filters are used. Additionally, in order to further improve the product recovery and/or to prevent filter clogging, a pre-filter can be used.
(16) Method according to any of the preceding items, wherein concentration step c) is carried out by applying a method selected from the group consisting of tangential flow filtration (TFF), ultracentrifugation and precipitation. The TFF is TFF ultrafiltration or TFF ultrafiltration and diafiltration in one step (TFF ultrafiltration/diafiltration).
   In one embodiment, when tangential flow filtration is used for step c), the molecular weight cut-off of the ultrafiltration membranes can for instance be equal to or less than 1000 kDa, preferably equal to or less than 750 kDa, more preferable equal to or less than 500 kDa, even more preferably equal to or less than 300 kDa, and most preferably 100-300 kDa. In a preferred embodiment, the molecular weight cut-off of the ultrafiltration membranes is not less than 50 kDa.
   When applying tangential flow filtration in step c), in a preferred embodiment, the volume reduction is in the range of 5-40 folds, preferably the volume reduction is more than 10 folds, more preferably the volume reduction is more than 15 folds. It is preferred that the volume reduction is not more than 30 folds. This may lead to improved results of the following treatment steps.
   In one embodiment, when ultracentrifugation is used for step c), the conditions of the ultracentrifugation can for instance be 30.000 g-200.000 g for more than 10 min. When applying ultracentrifugation in step c), in a preferred embodiment, the volume reduction of the ultracentrifugation is more than 10 folds, preferably more than 20 folds, more preferably more than 30 folds, even more preferably more than 40 folds.
   In one embodiment, if the concentration is carried out by applying precipitation, suitable chemicals are known to a person skilled in the art and can for instance be salt, methyl and ethyl alcohol and polyethylene glycol, in a respectively appropriate concentration. The suitable chemicals at an appropriate concentration precipitate the product, e.g. the viral antigen containing particles, but have no or minimal negative effect on said viral antigens. The chemicals are chosen such that they do not react with the (finally) desired product, e.g. the viral antigens. When applying precipitation in step c), in a preferred embodiment, the volume reduction is more than 10 folds, preferably more than 20 folds, more preferably more than 30 folds, even more preferably more than 40 folds.
(17) Method according to any of the preceding items, wherein in step d) the solubilization is carried out for a time period and at a temperature sufficient to solubilize essentially all of the enveloped viruses being present in the liquid harvest.
   In general, a time period and temperature sufficient to solubilize essentially all of the enveloped virus can be determined by a person skilled in the art. In one embodiment, the time period is between 0.2 h to 20 h at above 0°C, preferably at a temperature of 2°C to 30°C, preferably the time period is between 0.5 h and 6.0 h at a temperature of 2-25°C, and more preferably at a temperature of 2-8°C.
(18) Method according to any of the preceding items, wherein, if carried out, step e) is carried out by applying a method selected from the group consisting of ultracentrifugation and tangential flow filtration.
   In one embodiment, when ultracentrifugation is used in step e), the conditions of the ultracentrifugation for pelleting preferably are 30.000 g -200.000 g for more than 10 min., preferably more than 60.000 g for more than 30 min., more preferably more than 90.000 g for more than 30 min., even more preferably more than 120.000 g for more than 30min..
   In one embodiment, when tangential flow filtration is used in step e), the molecular weight cut-off of the ultrafiltration membranes preferably is more than 100 kDa and more preferably more than 300kDa. Further, the molecular weight cut-off of the membranes is preferably not larger than 500kD.
(19) Method according to any of the preceding items, wherein step f) is carried out by applying chromatography, preferably the chromatography is selected from the group consisting of ion exchange chromatography, hydrophobic interaction chromatography, affinity chromatography, mixed mode chromatography, pseudo affinity chromatography, liquid-liquid chromatography and size exclusion chromatography, more preferably the chromatography is selected from the group consisting of ion exchange chromatography, hydrophobic interaction chromatography, pseudo affinity chromatography, affinity chromatography and size exclusion chromatography.
(20) The method according to any of the preceding items, comprising an additional step of formulating the obtained viral antigen haemagglutinin and/or neuraminidase into a pharmaceutical composition.
(21) The method according to item (20), wherein the pharmaceutical composition is a vaccine preparation or an intermediate thereof, preferably the pharmaceutical composition is an enveloped virus vaccine, more preferably the pharmaceutical composition is an influenza virus vaccine.
(22) The method according to any of the preceding items, wherein step g) comprises the use of a composition having a conductivity in the range of between equal to or more than 3.0 to less than 5.0 mS/cm, preferably between equal to or more than 3.5 to equal to or less than 4.7 mS/cm, even more preferably in a range of between equal to or more than 3.6 and equal to or less than 4.65 mS/cm, when referenced to 25°C.
   In a preferred embodiment, step g) comprises a chromatography step, such as ion exchange chromatography, and the composition having the above defined conductivity is a buffer, for example a load buffer and/or an equilibration buffer comprising salt, suitable for use in combination with the respective chromatographic method used.
   The conductivity of the composition can be measured according to any suitable method that is known to a person skilled in the art, for instance by using electronic means such as a conductivity meter.
(23) The method according to any of the preceding items, wherein step g) comprises the use of a composition having a molar concentration in the range of between equal to or more than 1.0 M to equal to or less than 3.5 M, preferably in the range of between equal to or more than 1.0 M to equal to or less than 3.0 M, more preferably in a range of between equal to or more than 1.5 M to equal to or less than 3.0 M, and even more preferably in a range of between equal to or more than 1.5 M to equal to or less than 2.6 M, when referenced to 25°C. In a preferred embodiment, step g) comprises a chromatography step, such as hydrophobic interaction chromatography, and the composition having the above defined molar concentration is a buffer, for example a load buffer and/or an equilibration buffer comprising salt, suitable for use in combination with the respective chromatographic method used.
   The molar concentration can be measured by any suitable method that is known to a person skilled in the art.
   Using the composition as defined in items (22) and/or (23) can even further contribute to an improved method for the production of a viral antigen, in particular with respect to the viral antigen recovery.
(24) Use of a method for the manufacture of a vaccine preparation containing haemagglutinin and/or neuraminidase derived from a cell-based orthomyxovirus propagation culture, comprising the following steps, in the indicated order:
   i) reducing the volume of a liquid harvest containing enveloped virus obtained from a cell-based enveloped virus propagation culture, thereby obtaining a concentrated liquid harvest, and
   ii) solubilizing the orthomyxovirus being present in the liquid harvest by adding a detergent or a mixture of detergents comprising a cationic detergent to the concentrated liquid harvest obtained in step i),
   wherein step i) is followed directly by step ii), and
   whereby the reduction of the host-cell DNA being present in the vaccine preparation is at least 2.5 log, preferably at least 3.0 log, more preferably at least 3.5 log, even more preferably at least 4.0 log.
   The at least 2.5 log reduction of the host-cell DNA in the vaccine preparation is achieved by a combination of steps i) and ii) alone, at best additionally steps b), e), f) and/or g) of item (1). In any case, further DNA-specific steps, in particular DNA-digesting enzyme treatment steps and DNA-removing chromatography (affinity chromatography, gel chromatography and the like), and/or inactivation steps, do not necessarily or even do not at all have to be carried out in order to achieve the above defined reduction of host-cell DNA. If further DNA-specific steps, and/or inactivation steps, are carried out, these steps (and thus the whole method) can be carried out in a markedly improved way e.g. in terms of process economy, as e.g. a significant lower amount of DNA digestion enzyme or inactivation composition is necessary in order to achieve the above host-cell reduction.
   With regard to the terms "vaccine preparation", "viral antigen", "cell-based", "liquid harvest", "enveloped virus" and "detergent" reference is made to the specification.
   In a preferred embodiment, prior to step i), cell debris and/or microcarriers are removed from the liquid harvest containing enveloped virus.
(25) Use according to item (26), wherein steps i) and ii) correspond to steps c) and d) as defined in the above items.
(26) Use according to item (24) or (25), wherein prior to step i) steps a) and b) as defined in the above items and after step ii) steps e), f) and/or g) as defined in the above items are carried out.

### Detailed description of the invention

The present invention is now described in more detail by preferred embodiments and examples, which are however presented for illustrative purpose only and shall not be understood as limiting the scope of the present invention in any way. The invention in its broadest sense is as defined in the independent claims. Viruses that are used for the production of pharmaceutical compositions such as vaccine preparations or intermediates thereof can either be propagated in cell-based or egg-based systems. Dependent on the respectively used system, said pharmaceutical compositions are commonly classified either as egg-based or as cell-based compositions. For vaccine preparations or vaccines, the major requirement of the processing is the sufficient removal of impurities, such as, in the case of egg-based virus propagation systems, the embryonated-egg proteins such as ovalbumin, as well as the removal of non-product viruses, and in the case of cell-based virus propagation systems, during processing additional requirements such as significant reduction of host-cell DNA and of host-cell proteins has to be fulfilled. In the context of the present invention, it has been unexpectedly found that the combination of specific process steps, carried out in a defined order, provides an improved method compared to conventional methods, for instance with regard to the process itself, e.g. in terms of simplification the overall process, and/or with regard to the obtained products, e.g. in terms of quality, yield and/or purity. By carrying out the method according to the present invention, said beneficial and surprising effects can be obtained provided that the process comprises a volume reduction step of a liquid harvest containing the propagated viruses, resulting in the concentration of the virus (also referred to as virus particles) and other constituents in said liquid harvest, and a solubilization step that solubilizes the virus being present in the reduced volume of the liquid harvest. In particular, and distinct from prior art processes, a purification step, being present in the method according to the present invention, takes place only after the solubilization step has been completed, and, further, is directed to viral antigen rather than being directed to whole virus particles. Therefore, by applying the process according to the present invention, high product loss, laborious, time and cost-intensive purification step(s) intended for and targeted towards whole virus particle purification prior to solubilization can be omitted, yet obtaining compositions with satisfying purity and yield. Furthermore, the solubilization step is directly preceded by the volume reduction step. Without wishing to be bound by any theory, it is assumed that these steps, i.e. the solubilization step directly following the volume reduction step, can contribute to the beneficial effects, as the concentration step c) can increase the concentration of the host cell DNA, which, in turn, can be beneficial with regard to the precipitation efficiency.

Surprisingly, and distinct from conventional methods for the production of pharmaceutical compositions such as vaccines comprising viral antigen, the process according to the present invention represents an improved, robust process that allows obtaining an enhanced product recovery, while at the same time only requiring a reduced number of process steps compared to conventional processes. This reduced number of required process steps further contributes to an enhanced robustness of the process, as well as to a lowering of production costs and time conventionally needed for producing vaccine compositions. This is particularly useful in case of a pandemic or seasonal outbreak of an orthomyxovirus infection such as influenza, as there is a need for a fast production and release of pandemic or seasonal vaccines to the market, and all the more useful when vaccines are produced using egg-based culture systems for orthomyxovirus propagation, which is known to be very time consuming.
Moreover, with a decreasing number of process steps being applied to the pharmaceutical composition, the stress imposed on orthomyxovirus and viral antigens haemagglutinin and/or neuraminidase being present is reduced, which can lead to an improvement in efficacy and stability of the final product (e.g. the vaccine) and intermediates thereof.
Furthermore, by applying the method according to the present invention, despite the reduced number of process steps compared to conventional processes, vaccine preparations can be manufactured that fulfill the specific administrative requirements as to the tolerated maximum amount of protein and DNA contaminations. For instance, for influenza vaccine preparations the maximum amount of currently (2011) tolerated contaminations is 120 µg of non-haemagglutinin (HA) protein/dose and 10 ng of DNA/dose (in such reference, 1 dose corresponds to 500 µl vaccine preparation). A further advantage of the process according to the present invention is that it can be applied independent of the virus being propagated in a cell based or in an egg based system. Therefore, for instance if a cell-based system is used for orthomyxovirus propagation, the method according to the present invention provides for a significant reduction of host cell DNA contaminations, and if an egg-based system is used for virus propagation, the method provides for a reduction of contaminations resulting from the egg culture.
Due to the enhanced recovery of produced viral antigen haemagglutinin and/or neuraminidase, an increased number of vaccine doses may be prepared per production batch. This may for instance be particularly beneficial in case of high demands in vaccine preparations, as it is e.g. the case in seasonal influenza outbreaks.
As a typical and beneficial vaccine, the vaccine is an influenza subunit vaccine.
Furthermore, the process according to the present invention can cope with variations of virus strains, which means that the process is less strain-dependent. This is particularly useful when viruses are used that exhibit a constant drift and shift of the genome, such as influenza viruses.
All in all, the method according to the present invention can provide a remarkably improved method e.g. with regard to robustness, product recovery, production time and costs, and contaminations being present in the final product or intermediates thereof.
Therefore, in an aspect, the present invention relates to a method for the production of haemagglutinin and/or neuraminidase derived from orthomyxovirus, comprising the following steps, in the indicated order:
a) providing a fluid derived from a cell-based orthomyxovirus propagation culture or an egg-based orthomyxovirus propagation culture comprising said virus,
b) clarifying the fluid comprising orthomyxovirus by applying centrifugation, tangential flow filtration, or depth filtration, resulting in a liquid harvest comprising said virus,
c) reducing the volume of the liquid harvest obtained after step b) by a factor of at least 4 to obtain a concentrated liquid harvest, wherein this volume reducing step is carried out in one single step,
d) solubilizing the orthomyxovirus being present in the liquid harvest by adding a detergent or mixture of detergents comprising a cationic detergent to the concentrated liquid harvest obtained in step c), wherein the final concentration of said cationic detergent is at least 2000 µg/ml,
e) separating viral cores from the composition obtained after step d),
f) optionally removing cationic detergent from the composition obtained after step e),
g) purifying haemagglutinin and/or neuraminidase by applying chromatography,
wherein step c) is followed directly by step d),
and
wherein no purification step that is directed at specifically purifying orthomyxovirus is carried out prior to step d).
The viral antigen is an orthomyxovirus, such as influenza A, influenza B or influenza C.

In step a) of the method according to the present invention, a fluid is provided that comprises the above described orthomyxovirus. This fluid is derived from a cell-based orthomyxovirus propagation culture or an egg-based orthomyxovirus propagation culture. As it is known to a person skilled in the art, viruses, such as the above defined enveloped viruses, can be propagated in cell-based culture systems or in egg-based culture systems. Suitable cell lines used to grow viruses such as influenza viruses are typically of mammalian origin, and, thus, represent a mammalian cell line, preferably an animal cell line. Suitable animal cell lines are known to a person skilled in the art and may include cell lines of hamster, cattle, primate (including human and monkey) and dog origin. In certain embodiments, the cells of the mammalian cell line are selected from the group consisting of Vero, PerC6, BHK, 293, COS, PCK, MRC-5, MDCK, MDBK and WI-38, preferably the cells are MDCK cells. Viruses can be grown on cells according to any suitable method that is known to a person skilled in the art. For instance, viruses can be grown in adherent cell culture or in suspension cell culture. In one embodiment, the cells are grown on microcarriers. In a further embodiment, the cells can also be adapted to grow in suspension. For cell-based culture systems, any suitable medium can be used. In a preferred embodiment, cells are cultivated in serum-free and/or protein-free medium. For cell-based virus propagation, usually, the virus or live virus preparation, respectively, is inoculated into the cell culture where it multiplies. The virus propagation can be done in a batch wise, or a feed-batch wise, or a perfusion wise. After 2-5 days at 25-37°C, the virus propagation is usually stopped. The virus containing fluid contained in the virus propagation bioreactor can be fed directly into its purification process or transferred into a separated vessel. Then the virus containing fluid in the separated vessel is fed into a vaccine purification process.

Alternatively, viruses can be propagated in egg-based systems. In an egg-based manufacturing, usually the working seed virus is injected into embryonated hen's eggs. The viruses start to propagate in the allantoic fluid (egg-white) of the egg to increase the amount of virus. After two-to three days of incubation, the eggs are chilled to 2-8°C and the allantoic fluid of the individual eggs is harvested and polled using manual or automated vacuum harvest systems. The collected virus containing allantoic fluid is the egg-based fluid comprising virus and is further processed in the subsequent purification process.

Depending on the culture system used for virus propagation, the fluid comprising the propagated viral particles may contain different contaminants. For instance, potential contaminants being present in egg-based culture are egg-based protein such as egg albumin, or antibiotics, whereas a typical contamination being present in cell-based cultures is residual host-cell DNA. Advantageously, the method according to the present invention is not restricted with regard to the system used for orthomyxovirus propagation; rather, the beneficial effects can be reached when using fluid comprising orthomyxoviruses propagated in cell-based culture systems and egg-based culture systems as well.

The fluid in step a) can produced by using a disposable bioreactor. By using such a disposable bioreactor, for instance the obtained product itself can be improved e.g. in terms of quality and/or purity. This is in particular beneficial if the method is carried out in large-scale, e.g. in industrial scale.

The fluid comprising the orthomyxovirus is subjected to a clarification step. The clarification step is carried out on the fluid comprising virus particles, however not on sedimented or otherwise pelleted matter. Within the meaning of the present invention, it is the purpose of this clarification step to remove particulate matter from the fluid, such as cell debris, protein impurities and/or support material used for growing cells, such as microcarriers. Within the meaning of the present invention, the clarification step e.g. removes the particulate matter from the fluid, but retains more than 80 % of viral antigens of the orthomyxovirus. Protein impurities can for instance be allantoin, collagen and/or albumin such as ovalbumin. For clarification, any suitable means or method that is known to a person skilled in the art can be used. In the present invention, tangential flow filtration (TFF), depth filtration, or centrifugation are applied. Thereby, the settings of each of the respectively applied clarification means (TFF, depth filtration, centrifugation) are chosen in such a way that the above indicated purpose is reached. In general, and given this purpose, said settings for each of the respective means are known to a person skilled in the art. In a preferred embodiment according to the present invention, when applying centrifugation, a centrifugal force of equal to or less than 10000 g can be applied for a time period of equal to or less than 2 minutes (min.). Applying less centrifugal force for a time period of equal to or less than 2 minutes in this clarification step, e.g. equal to or less than 1000 g, preferably equal to or less than 500 g, more preferably equal to or less than 300 g, even more contributes to beneficial effects of the present invention, for instance it can contribute to a further enhanced recovery of viral antigen. As to further parameters when applying centrifugation, as well as with regard to the parameters for tangential flow filtration or depth filtration, reference is made to elsewhere in this specification.

Within the meaning of the present invention, the obtained clarified fluid is denoted as "liquid harvest".

A key aspect of the method according to the present invention is the reduction of the volume of the clarified fluid containing the propagated orthomyxovirus particles, directly followed by a solubilization/detergent treatment step of the orthomyxoviral particles being present in the volume-reduced, concentrated, liquid harvest. It has been surprisingly found that this volume reduction, directly followed by treating the orthomyxovirus particles with detergent as described elsewhere herein, leads to an improved recovery and thus improved yield of viral antigen haemagglutinin and/or neuraminidase. Furthermore, it has been surprisingly found that if orthomyxovirus propagation has been carried out with cell-based systems, an unexpected, enhanced reduction of host-cell DNA can be reached.

Within the meaning of the present invention, the term "directly followed" denotes that e.g. no treatment steps or no special, active purification steps are carried out in between steps c) and d). This means that e.g. no treatment steps are carried out to the liquid harvest between steps c) and d), for instance no inactivation or purification step is carried out. This can also mean that no further reagents are taken from or added to the liquid harvest between steps c) and d). In another aspect, step b) is directly followed by step c) which is directly followed by step d). This can further contribute to an enhancement of the method of the present invention, e.g. in terms of simplification, or in terms of time efficiency and cost reduction, while still remaining high product (viral antigen) recovery, yield and/or purity.

Inactivation leads to the removal of the infectivity of virus and is typically carried out by treating viruses with an effective amount of one or more of the following chemical means: Detergents, formaldehyde, β-propiolactone, methylene blue, psoralen, carboxyfullerene (C60), binary ethylamine, acetyl ethyleneimine or combinations thereof. Further methods of inactivation are known to a person skilled in the art and comprise for instance treating virus with physical means, such as gamma irradiation, and/or UV light.
In certain embodiments of the present invention, no inactivation by applying the same detergent which is used for solubilizing the orthomyxovirus (step d)) is carried out.
In further embodiments according to the present invention, no inactivation, particularly no inactivation including treatment of orthomyxovirus with formaldehyde, β-propiolactone and/or UV light (such as UV-C), preferably with formaldehyde and/or β-propiolactone, is carried out prior to step d). In another aspect it is possible to apply a step of inactivation of the orthomyxoviruses after the step of solubilization of the orthomyxovirus, i.e. after step d). In an even further aspect, no inactivation step at all is carried out during steps a) to f). This can further contribute to an enhancement of the method according to the present invention, e.g in terms of costs and/or work. Sometimes, β-propiolactone is also used for inactivation of residual host-cell DNA that can be present if virus propagation has been carried out on cell-based systems. However, β-propiolactone is an alkylating agent that not only alkylates DNA but also the product antigens (viral antigens), and might therefore negatively influence the quality, e.g. in terms of stability and/or immunogenicity, of said antigens. Therefore, it is an advantage of the method according to the present invention, that, surprisingly, despite the omission of inactivation agents such as β-propiolactone, the limits of maximal allowed host-cell DNA impurities in the final product (such as vaccine preparation) can be complied with.

The concentration of the liquid harvest in step c) can be achieved by suitable methods. Said methods are directed at reducing the volume of the liquid harvest. It is not the purpose of this step to specifically purify orthomyxovirus particles, i.e. this step is not a main purification step. Rather, this step aims at reducing the volume of the liquid harvest comprising the orthomyxovirus particle to obtain a concentrated liquid harvest.

During the step of reducing the volume, it may happen that small impurities are separated from the liquid viral harvest. Small impurities are any matter such as molecules, particles, or medium components, having a size that is below that of the enveloped virus, preferably below about 1000 kDa, further preferred below about 500 kDa, even further preferred below about 300 kDa. This separation of small impurities, if it takes place, is only based on the size of the separated impurities, however not on the density of the impurities. A separation of impurities that is not only based on the size of said impurities, however also on the density of said impurities, takes place when carrying out purification methods that comprise gradients, such as gradient centrifugations. An example of a gradient centrifugation is the sucrose gradient ultracentrifugation. The step of reduction of volume e.g. contributes to obtaining an improved recovery of viral antigens and/or improved removal of host-cell DNA.

In a preferred embodiment, the volume can be reduced by applying TFF ultrafiltration or TFF ultrafiltration and diafiltration in one step (TFF ultrafiltration/diafiltration), ultracentrifugation or precipitation, preferably TFF ultrafiltration is used in order to reduce the volume. Thereby, the settings of each of the respectively applied concentration means (TFF, TFF ultrafiltration/diafiltration, ultracentrifugation or precipitation) are chosen in such a way that the above indicated purpose, i.e. the reduction of the volume and the concentration of the virus particles, is reached. In a preferred embodiment according to the present invention, the volume reduction is reached by applying TFF ultrafiltration/diafiltration using 50 kDa-1000 kDa cut-off membranes, preferably 100 kDa-300 kDa cut-off membranes, and preferably not less than 50 kDa.

In one embodiment, when ultracentrifugation is used to reduce the volume, the conditions of the ultracentrifugation can for instance be 30.000 g-200.000 g of RCF for more than 10 min.
In one embodiment, if the concentration is carried out by applying precipitation, suitable chemicals are known to a person skilled in the art and can for instance be salt, methyl and ethyl alcohol and polyethylene glycol, in a respectively appropriate concentration. The suitable chemicals at an appropriate concentration precipitate the product, e.g. the viral antigen containing particles, but have no or minimal negative effect on said viral antigens. The chemicals are chosen such that they do not react with the desired antigens haemagglutinin and/or neuraminidase, i.e. the orthomyxoviral antigens and do not alter the immunogenicity of the desired antigens.
Also preferred, the step of reduction of volume in step c) is not carried out by using a density gradient centrifugation method such as sucrose gradient ultracentrifugation. A gradient ultracentrifugation is a high-cost and labour-intensive process step that requires a high level of maintenance, and the operation is expensive. Therefore, by omitting gradient ultracentrifugation step, the process can be enhanced for instance in terms of reduced production costs and labour.

In one embodiment of the present invention, the volume is reduced by a factor of at least 5, or at least 9, also preferably at least 12, also preferably at least 13, or at least 15, and also preferably at least 20, or at least 25. A typical maximum volume reduction is 30, 35, 40 or 45. It has been found in the present invention that reducing the volume by a factor as indicated herein, in particular in combination with a detergent in a concentration as indicated below, further contributes to an enhancement of the recovery of viral antigen haemagglutinin and/or neuraminidase. Moreover, if a cell-based system is used for orthomyxovirus propagation, an unexpectedly enhanced reduction of host-cell DNA can be reached, e.g. a reduction of at least 2.5 log.
This reduced, concentrated volume comprising orthomyxovirus particle is directly subjected to a solubilization step. The viral antigens haemagglutinin and/or neuraminidase can be present in different forms such as large particles either in aggregates and/or attached to cell and cell debris, filamentous form virus, intact and free spherical virus particles, virus fragments and loose viral antigens which are non-virus associated. The solubilization step is carried out in order to free the viral antigens haemagglutinin and/or neuraminidase from the different structures.

Solubilizing the orthomyxovirus being present in the liquid harvest is carried out by adding a detergent, or mixture of detergents, comprising, or consisting of, a cationic detergent to the liquid harvest obtained after step c), wherein the final concentration if said cationic detergent is at least 2000 µg/ml. Cationic detergents are known to a person skilled in the art and are e.g. based on quaternary ammonium cations like cetyltrimethylammonium bromide or other alkyl trimethylammonium salts, alkyl amine salts like stearyl amine acetate or coconut alkyl amine acetate, benzalkonium chlorides and bromides, for example benzethonium chloride or methylbenzethonium chloride, stearylaminepolyglycolether or oleylaminepolyglycolether, wherein CTAB (cetyl trimethylammonium bromide) is preferred. Further preferred, a mixture of at least two detergents is used. Preferably, the mixture of detergents comprises, or consists of, cationic and non-ionic detergents. Preferably, the mixture of detergents comprises or consists of a cationic detergent, preferably CTAB, and a non-ionic detergent, like alkylpolyethylene oxid, alkyl polyglycoside, including: octyl glycoside and decyl maltoside, e.g. nonidet P10 or nonidet P40 surfactants, MEGA-8, -9 or -10, Triton X 100 and related detergents or polysorbates such as detergents of the Tween family, like Tween 20, Tween 40, Tween 60, Tween 80, APO-10, APO-12, C8E6, C10E6, C12E6, C12E8, C12E9, C12E10, C16E12, C16E21, Heptane-1,2,3-triol, lubrol PX, genapol family, n,-Dodecyl-b-D-glucopyranoside, thesit, pluronic family, etc.; preferably a polysorbate such as polysorbate 80, is used. Multiple detergents may be added as a premix (which means that they are present in a mixture) or are added separately, but at the same time or directly one after another. It is preferred not to carry out a detergent exchange where a first detergent is removed prior the addition of a second detergent.
The total amount, i.e. the final concentration, of cationic detergent(s), preferably CTAB, is high, which means that is higher than the final amount typically being used for solubilization purposes. Therefore, the final concentration is at least about 2000 µg/ml, also preferred at least about 2500 µg/ml, also preferred at least about 3000 µg/ml, also preferred at least about 3500 µg/ml, also preferred at least about 4000 µg/ml, also preferred at least about 4500 µg/ml, also preferred at least about 5000 µg/ml, also preferred at least about 5500 µg/ml, and also preferred at least about 6000 µg/ml. A possible maximum amount of cationic detergent(s) is about 8000 µg/ml, also preferred about 7000 µg/ml, also preferred about 6000 µg/ml. Particularly preferred ranges are about 4000 µg/ml - about 6000 µg/ml, about 4500 µg/ml - about 6000 µg/ml, about 4000 µg/ml - about 5000 µg/ml, about 4500 µg/ml - about 5000 µg/ml.

The total amount, i.e. the final concentration, of non-ionic detergent(s), preferably polysorbate, is at least about 300 µg/ml, also preferred at least about 500 µg/ml, also preferred at least about 800 µg/ml, also preferred at least about 900 µg/ml, also preferred at least about 1000 µg/ml, also preferred at least about 1200 µg/ml, also preferred at least about 1400 µg/ml, also preferred at least about 1800 µg/ml. A preferred maximum amount of non-ionic detergent(s) is about 4000 µg/ml, also preferred about 3000 µg/ml, also preferred about 2000 µg/ml. Particularly preferred ranges are about 300 µg/ml - about 1500 µg/ml, about 300 µg/ml - about 1100 µg/ml, about 900 µg/ml - about 1500 µg/ml, about 900 µg/ml - about 1200 µg/ml.

In a further embodiment, the liquid viral harvest is reduced by a factor of at least 20 at the presence of a final concentration of cationic detergent of at least 5000 µg/ml, or the harvest is reduced by a factor of at least 15 at the presence of a final concentration of cationic detergent of at least 4000 µg/ml, or the harvest is reduced by factor of at least 10 at the presence of a final concentration of cationic detergent of at least 2500 µg/ml.
By applying these conditions, particularly beneficial results with regard to product recovery can be achieved. Moreover, if a cell-based system is used for orthomyxovirus propagation, in addition to the enhanced product recovery, an enhanced reduction of host-cell DNA impurities can be achieved.

The solubilization can be carried out at conditions that are suitable in order to achieve the solubilization of essentially all, preferably all, orthomyxoviral particles being present in the concentrated liquid harvest. In a preferred embodiment, the solubilization is carried out by incubating the concentrated viral harvest between 0.2 h to 20 h at above 0°C, preferably at a temperature of 2°C to 30°C, preferably between 0.5 h and 6.0 h at a temperature of 2-25°C, more preferably at a temperature of 2-8°C.

When using a cell-based system for orthomyxovirus propagation, it is preferred not adding nuclease, e.g. a DNase, such as Benzonase®, prior to step d), or during all steps a) to f). In the prior art, the fluid comprising virus or the liquid harvest is treated with nuclease, and/or by β-propiolactone (BPL), and/or by zonal ultracentrifugation and/or by carrying out chromatography steps in order to degrade and/or remove nucleic acids of the host cells, and, thus, to minimize host-cell DNA impurities. For instance, for influenza-based purification processes, often more than 4 log reduction of host-cell DNA has to be achieved in order to fulfil the administrative regulations. It has now surprisingly found that by applying the inventive method, a sufficiently high reduction of host-cell DNA for fulfilling the administrative regulations can be achieved, even without nuclease treatment such as Benzonase® treatment. This significantly enhances the process in terms of cost efficiency and time. Furthermore, BPL treatment is not required and can be limited to desired cases, if any. In certain embodiments, no BPL treatment is carried out prior to step d), in certain embodiments no BPL treatment is carried out at all.

After step d), a step e) of separating the viral cores obtained after step d) from the composition can be carried out. The separation of the viral cores can be carried out by any suitable method that results in said desired separation.
In an embodiment of the present invention, this separation step is carried out by pelleting off the viral cores, e.g. by ultracentrifugation (however in a preferred embodiment not by density gradient ultracentrifugation). The viral surface proteins, e.g. the influenza viral (surface) proteins such as HA, NA (neuraminidase) or M protein, stay in the supernatant and can be subjected to further process steps in order to finally arrive at subunit virus vaccines. For example, centrifugation conditions chosen in order to pellet out the viral cores are known to a person skilled in the art. For instance, the ultracentrifugation can either be in batch-wise or in continuous-wise, and the ultracentrifugation conditions used are from 30.000 g for more than 10 minutes (min.), to 200.000 g for more than 10 min., respectively at a suitable temperature, such as at 2-25°C, more preferably at 2-8°C. In one embodiment, the ultracentrifugation conditions for pelleting out the obtained viral cores are 100.000 g for 30 min. at a temperature range of 2-8°C.

In a further embodiment, after step d) and prior to step g), detergent derived from step d) can be removed. This detergent removal can be carried out by any method that is suitable for this purpose, for instance by Amberlite treatmentment or TFF. When using Amberlite treatment, and after step e) has been carried out, in a preferred embodiment, the Amberlite is added to the supernatant collected after having pelleted out the viral cores at a ratio of the mass of Amberlite to the mass of the cationic detergents about 100:1, incubated at a suitable temperature such as a temperature being in the range of between 2-8°C, for a suitable time, e.g. for 8 to 24, suitably for 16 hours. Then, the Amberlite is removed by any suitable method that is known to a person skilled in the art, such as by using conventional flow filtration/dead end filtration, sieves, or TFF. When a filter is used, it should be chosen in such a way that it is capable of retaining particles that are bigger than 5 µm or bigger than 7 µm. Further, the Amberlite can be removed by using sieves. The opening of appropriate sieves is in the range of 30-200 µm, preferably less than 150 µm, more preferably less than 100 µm, even more preferably around 50 µm. In one embodiment, for the removing of the detergent(s), no chromatography is used.

Finally, in step g), which represents the main purification step or main purification method, respectively, orthomyxovirus haemagglutinin and/or neuraminidase antigen is purified.

The purification of the viral antigen haemagglutinin and/or neuraminidase can be carried out by any method that is suitable for specifically purifying viral antigen from the composition obtained after step e) or, if step f) has been carried out, after step f). Preferred purification methods are purification methods such as chromatography methods, e.g. ion exchange chromatography, hydrophobic interaction chromatography, pseudo-affinity chromatography, affinity chromatography, size exclusion chromatography, liquid-liquid chromatography, and the like. In one embodiment, preferred purification methods are ion exchange chromatography and hydrophobic interaction chromatography.
The process of the present invention can be used as a part of an overall production process of preparing an orthomyxovirus subunit vaccine preparation, preferably an influenza subunit vaccine.
The present invention further refers to the method according to the present invention wherein the method is used in influenza subunit vaccine production.

The present invention further refers to a method as described herein, further comprising an additional step of formulating the obtained, purified viral antigen haemagglutinin and/or neuraminidase into a pharmaceutical composition, wherein, preferably, the pharmaceutical composition is a vaccine preparation or an intermediate thereof. In a particularly preferred embodiment, the pharmaceutical composition is an influenza vaccine. This additional step(s) is (are) (a) step(s) that may have to be carried out in order to arrive at pharmaceutical compositions. Such step can for instance be a virus inactivation step such as an irradiation step, e.g. an UVC irradiation step. In general, such a step ensures that the viral antigen is inactive and free of active contaminant viruses. To minimize microbe contamination, e.g. a bioburden reduction filtration might have to be carried out. Furthermore, for instance if the viral antigen has to be formulated into a virus vaccine, it might be that adjuvant(s) has (have) to be added in a certain concentration. A further additional step may be the addition of further viral antigen, such as viral antigen being derived from another virus strain. A further additional step may be the addition of buffer, and/or the inclusion of preservative. A further additional step may be a clearance step, or a sterile filtration step. The term "pharmaceutical composition" denotes a composition that can be used in, or on, the body to prevent, diagnose, alleviate, treat or cure a disease in humans or animals. Preferably, such a pharmaceutical composition is a vaccine preparation or an intermediate product thereof, more preferably an enveloped virus vaccine preparation such as an influenza virus vaccine preparation, incl. e.g. a seasonal, a pandemic or an epidemic influenza virus vaccine preparation. Examples of a pandemic or epidemic influenza virus vaccine preparation are an avian, a human or a pig influenza virus vaccine preparation.

The present invention also refers to a method for the manufacture of a vaccine preparation containing haemagglutinin and/or neuraminidase derived from a cell-based orthomyxovirus propagation culture, comprising steps a) to g) of any of the preceding claims, and a step of formulating the obtained, purified haemagglutinin and/or neuraminidase into a vaccine preparation.

With regard to the terms "vaccine preparation", "viral antigen haemagglutinin and/or neuraminidase", "cell-based", "liquid harvest", "orthomyxovirus" and "detergent" reference is made to the specification.

It has been surprisingly found that by using the above method in the manufacture of a vaccine preparation, the host-cell DNA contamination can be significantly reduced.

### Description of the figures

**Fig. 1****:** This figure shows the effect of the concentration of CTAB and the overall concentration factor, i.e. the final concentration of the harvest after the detergent CTAB has been added, on the recovery of the major surface antigen HA. Fig. 1 represents an example of many results and graphs.
**Fig. 2****:** This figure shows the effect of the concentration of CTAB and the overall concentration factor, i.e. the final concentration of the harvest after the detergent CTAB has been added, on DNA reduction. Fig. 2 represents an example of many results and graphs.

### Examples

### Example 1

This example demonstrates how to process influenza virus harvest into the partially purified intermediate of the viral surface antigens Hemagglutinin (HA) and Neuraminidase (NA) with minimal product loss and with more than three log reduction in host-cell DNA. The obtained intermediate will further be purified by using chromatography.

A general overview of the process steps is given in the following work flow:

| | |
|---|---|
| 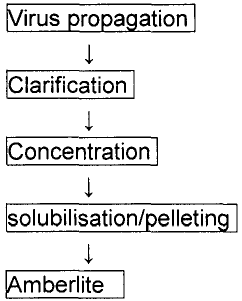 | Embryonated hens' eggs or in mammalian cells |
| | centrifugation, Tangential flow filtration (TFF), or depth filtration |
| | TFF, ultracentrifugation, or precipitation |
| | Detergents followed by ultracentrifugation or TFF |
| | Keep or remove detergents prior to HA& NA purification |

Influenza virus was propagated in MDCK cells. The harvests were clarified by centrifugation at 3220 g for 8 min. The clarified harvest was concentrated about 4-13.7 fold by TFF using 100KD-300 kD cassette. The clarified and concentrated harvest was solubilized using 2400-3000 µg/ml of CTAB and 600-90 µg/ml Tween-80 at 2-8°C. The clarified, concentrated, and solubilized harvest was centrifuged at 100.000 g for 30min at 2-8°C. The supernatant were collected. Amberlite was added into the collected supernatant at the ratio of the mass of Amberlite to the mass of CTAB of 100:1. After incubated at 2-8°C for 16 h, the added Amberlite was removed by filtering the suspension through 5 µm filters. The filtrate is the partially purified intermediate of the viral surface antigens Hemagglutinin (HA) and Neuraminidase (NA). HA was measured by Single Radial immune Diffusion (SRD) and DNA was measured by quantitative real time polymerase chain reaction (qPCR).
The results are depicted in the following:
HA recovery of A/Uruguay X-175C, clarification at 3220 g for 8 min, 10x concentrated using 300 kDa cassette, solubilized at 900 µg/ml Tween-80 and 3000 µg/ml CTAB, centrifuged at 100.000 g for 30 min, and Amberlite treated harvest. After corrected for the dilution introduced during the solubilization, the overall concentration factor is 4x :

| Sample | HA [µg/ml] | Total HA recovery [%] |
|---|---|---|
| Harvest | 32 | |
| Filtrate of clarified, 10x concentrated, solubilized, centrifuged, Amberlite treated, harvest. Overall concentration factor is 4 | 120 | 92% |

HA recovery of B/Malysia/2506/2004WT, clarification condition: 300 g for 2 min. , 4x concentrated using 100 kDa cassette, solubilized at 600 µg/ml Tween-80 and 2400 µg/ml CTAB, centrifuged at 100.000 g for 30 min, and Amberlite treated harvest. After corrected for the dilution introduced during the solubilization, the overall concentration factor is 1.8x.

| Sample | HA [µg/ml] | Total HA recovery [%] |
|---|---|---|
| Harvest | 25 | 100% |
| Clarified and 4x concentrated (100 kDa) harvest | 93.5 | 94% |
| Filtrate of clarified, concentrated, solubilised, centrifuged; Amberlite treated harvest. The overall concentration factor is 1.8x. | 42 | 93% |

HA recovery of A/California X-179A, Clarification condition: 300 g for 2 min, 13.7x concentrated using 100 kDa cassette, solubilization: 900 µg/ml Tween-80 and 3000 µg/ml CTAB, centrifuged at 100.000 g for 30 min, and Amberlite treated harvest. After corrected for the dilution introduced during the solubilization, the overall concentration factor is 4.8x:

| Sample | HA [µg/ml] | Total HA recovery [%] | DNA [ng/ml] | DNA Log Reduction |
|---|---|---|---|---|
| Harvest | 25 | 100% | 72513 | 0 |
| Clarified and 13.7x concentrated (100KD) Harvest | 292 | 85% | 42187 | 1.4 |
| Filtrate of clarified, concentrated, solubilised, centrifuged, Amberlite treated harvest, the overall concentration factor is 4.8x. | 112 | 93% | 53 | 3.8 |

### Example 2

Example 2 demonstrated that the HA and NA antigens of the filtrate of clarified, concentrated, solubilized, Amberlite harvest can be purified sufficiently by ion exchange chromatography so that its total protein (TP)/100 µg HA is lower than 240 µg/100 µg HA and its host-cell DNA is less than 20 ng/100 µg HA. NA antigen are co-purified together with HA antigens.

Influenza virus was propagated in MDCK cells. The harvests were clarified by centrifugation at 300 g for 2 min or 3220 g for 8 min. The clarified harvests were concentrated about 10 fold by TFF using a 300 kD cassette. The clarified and concentrated harvests were solubilized using 3000 µg/ml of CTAB and 900 µg/ml Tween-80 at 2-8°C. The clarified, concentrated, and solubilized harvests were centrifuged at 100.000 g for 30 min. at 2-8°C .The supernatants were collected. Amberlite was added into the collected supernatant at a ratio of the mass of Amberlite to the mass of CTAB of 100:1. After incubated at 2-8°C for 16 h, the added Amberlite was removed by putting the suspension through 5 µm deep filters. The filtrate was conditioned before loaded onto Capto® Q chromatography columns. After washing with loading buffers, the HA and NA antigens were eluted. Flow through, wash, and elution were fractioned. Based on UV signal, several fractions were selected for HA-SRD, NA, DNA, total protein analysis. The results are indicated below:

**Capto Q**

| strain | Conductivity of load & Equilibration buffer | pH of load & Equilibration buffer | HA recovery of elution fraction collected | Total protein µg/100 µg HA | DNA ng/ 100 µg HA | NA |
|---|---|---|---|---|---|---|
| A/California X-179A | 6 | 8 | 45% | 104 | 4 | Not measured |
| A/Uruguay X-175C | 5 | 7.3 | 55% | 208 | | Not measured |
| A/California X-179A | 4.65 | 8 | 92% | 225 | ≥ 1 | present |
| A/California X-179A | 3.6 | 8 | 91% | 189 | ≥ 1 | present |
| A/California X-179A | 3.9 | 8 | 85% | 189 | ≥ 1 | present |

As seen, HA recovery of A/California was influenced by the conductivity. When the conductivity is in the range of 3.6-4.65 mS/cm, HA recovery was between 85 %-92 %. Taking into account of the HA recovery of the preparation of the load of the chromatography step, the clarified, concentrated, solubilized, and Amberlite steps, the total recovery of HA can be about 80 % for A/California. NA was co-purified together with HA antigens. The content of host-cell DNA and TP met their specification.

For A/Uruguay, the HA recovery was 55 % when the conductivity was 5 mS/cm at pH 7.3. Taking into account of the HA recovery of the preparation of the load of the chromatography step, the clarified, concentrated, solubilised, and Amberlite steps, the total recovery of HA can be about 50 % for A/Uruguay. NA antigen was not measured. The content of TP met its specification. The host-cell DNA was not measured. By lowering the conductivity as A/California, HA recovery of A/Uruguay was expected to increase as well.

### Example 3

Example 3 demonstrated that the HA and NA antigens of the filtrate of clarified, concentrated, solubilized, Amberlite harvest can be purified sufficiently by hydrophobic interaction chromatography so that its total protein/100 µg HA is lower than 240 µg/ml and its host-cell DNA content is less than 20 ng/100µg HA. NA antigen is co-purified together with HA antigens.

The preparation of the clarified, concentrated, solubilized, Amberlite harvest is the same as that of the example 2. The filtrate was conditioned before loaded onto hydrophobic interaction chromatography (HIC) columns. After washing with loading buffers, the HA and NA antigens were eluted. Flow through, wash, and elution were fractioned. Based on UV signal, several fractions were selected for HA-SRD, NA, DNA, total protein analysis. The results are indicated below:

**HIC**

| strain | Salt Concentration of load & Equilibration buffer | pH of load & Equilibration buffer | HA recovery of elution fraction collected | Total protein µg/100 µg HA | DNA ng/ 100µg HA | NA |
|---|---|---|---|---|---|---|
| A/California X-179A | 2.3M | 7.4 | 68% | 152 | 19 | present |
| A/Uruguay X-175C | 2.6M | 7.4 | 91% | 88 | 15 | present |
| B/Malaysia | 1.5M | 7.4 | 73% | 202 | 8 | present |
| B/Malaysia | 1.7M | 7.4 | 68% | 118 | 15 | present |
| B/Malaysia | 2.0M | 7.4 | 71% | 180 | 9 | present |

As seen, HA recovery of A/California of hydrophobic interaction chromatography was 68 %-91 % under the tested conditions. Salt concentration influenced the HA recovery. Taking into account of the HA recovery of the preparation of the load of the chromatography step, the clarified, concentrated, solubilized, and Amberlite steps, the total recovery of HA was between 60-80 % for A/California. NA was co-purified together with HA antigens. The content of host-cell DNA and TP met their specification.

For B/Malaysia, the HA recovery was around 70 % when the salt concentration used was 1.5-2.0M at pH 7.4. Taking into account of the HA recovery of the preparation of the load of the chromatography step, the clarified, concentrated, solubilized, and Amberlite steps, the total recovery of HA was 60 % for B/Malaysia. NA antigen was co-purified together with HA antigens. The content of host-cell DNA and TP met their specification.

### Example 4

### Method and materials

Influenza virus was propagated in MDCK cells. The harvests were clarified by centrifugation at 300 g for 2 min or 3220 g for 8 min. The clarified harvests were concentrated different folds by TFF using a 100-300 kDa cassette. The clarified and concentrated harvests were solubilized using 600 - 3000 µg/ml of CTAB and 300 - 900 µg/ml Tween-80 at 2-8°C. The clarified, concentrated, and solubilized harvests were centrifuged at 100.000 g for 30 min. at 2-8°C.The supernatants were collected. Amberlite was added into the collected supernatant at a ratio of the mass of Amberlite to the mass of CTAB of 100:1. After incubation at 2-8°C for 16 h, the added Amberlite was removed by putting the suspension through 5 µm deep filters. The filtrate was measured for HA-SRD (single radial immunodiffusion assay), NA, DNA, and a total protein analysis was carried out.

### Example 4.1

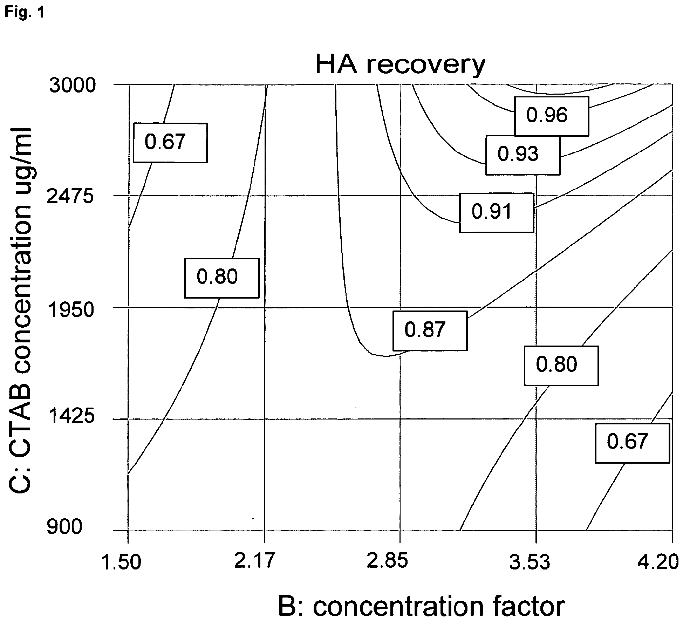

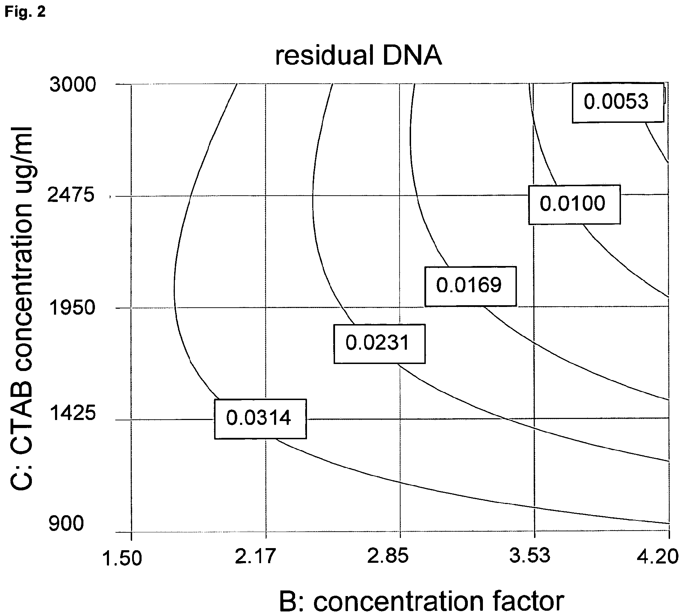

This example demonstrates that the combination of a high concentration factor and a high concentration of cationic detergent is preferable both for the recovery of the antigen and for the removal of host-cell DNA. The strain used was B/Brisbane. After the clarification and concentration, the harvest was incubated at the presence of detergents at different conditions. The concentrations of the detergents, the incubation time, and the concentration factors were varied. The effect of the concentration of CTAB and the concentration factors on the recovery of the major surface antigen HA and the DNA reduction was shown in the Figure 1 and Figure 2. As can be seen, the increase of the concentration of cationic detergents and the concentration factor is beneficial both for the recovery of products (Fig. 1) and for the reduction of host-cell DNA (Fig. 2).

### Example 4.2

This example demonstrates that both high product recovery of B/Florida/4/2006 and significant reduction of host cell DNA can be realized by using 3000 µg/ml of CTAB and 900 µg/ml of Tween-80 and followed by centrifugation at 100.000 g for 30 min.

| Sample and description | DNA [ng/ml] | DNA Log Red | SRD [µg/ml] | HA recovery | Remark |
|---|---|---|---|---|---|
| Clarified and 8.4x concentrated Harvest | 26349 | | 42 | | |
| Solubilized at 3000 µg/ml CTAB & 900 µg/ml Tween 80, centrifuged, and Amberlite treated | 1 | 4.0 | 23 | ≥ 95% | 2.5 x diluted |

### Example 4.3

This example demonstrates that both high product recovery of A/Wisconsin and significant reduction of host cell DNA can be realized by using 3000 µg/ml of CTAB and 900 µg/ml of Tween-80 and followed by centrifugation at 100.000 g for 30 min.

| Sample and description | DNA [ng/ml] | DNA Log Red | SRD [µg/ml] | HA recovery | Remark |
|---|---|---|---|---|---|
| Clarified and 8.4x concentrated Harvest | 32895 | | 258 | | |
| Solubilized at 3000 µg/ml CTAB & 900 µg/ml Tween 80, centrifuged, and Amberlite treated | 2 | 3.8 | 97 | 94% | 2.5 x diluted |

### Example 4.4

This example demonstrates that both high product recovery of A/Victoria and significant reduction of host cell DNA can be realized by using 3000 µg/ml of CTAB and 900 µg/ml of Tween-80 and followed by centrifugation at 100.000 g for 30 min.

| Sample and description | DNA [ng/ml] | DNA Log Red | SRD [µg/ml] | HA recovery | Remark |
|---|---|---|---|---|---|
| Clarified and 10.2x concentrated Harvest | 37417 | | 185 | | |
| Solubilized at 3000 µg/ml CTAB & 900 µg/ml Tween 80, centrifuged, and Amberlite treated | 5 | 3.5 | 87 | ≥ 95% | 2.5 x diluted |

### Example 4.5

This example demonstrates that both high product recovery of A/California and significant reduction of host cell DNA can be realized by using 3000 µg/ml of CTAB and 900 µg/ml of Tween-80 and followed by centrifugation at 100.000 g for 30 min.

| Sample and description | DNA [ng/ml] | DNA Log Red | SRD [µg/ml] | HA recovery | Remark |
|---|---|---|---|---|---|
| Clarified and 13.7x concentrated Harvest | 42187 | | 292 | | |
| Solubilized at 3000 µg/ml CTAB & 900 µg/ml Tween 80, centrifuged, and Amberlite treated | 53 | 2.5 | 112 | 96% | 2.5 x diluted |

## Claims

1. A method for the production of haemagglutinin and/or neuraminidase derived from orthomyxovirus, comprising the following steps, in the indicated order:
a) providing a fluid derived from a cell-based orthomyxovirus propagation culture or an egg-based orthomyxovirus propagation culture comprising said virus,
b) clarifying the fluid comprising orthomyxovirus by applying centrifugation, tangential flow filtration, or depth filtration, resulting in a liquid harvest comprising said virus,
c) reducing the volume of the liquid harvest obtained after step b) by a factor of at least 4 to obtain a concentrated liquid harvest, wherein this volume reducing step is carried out in one single step,
d) solubilizing the orthomyxovirus being present in the liquid harvest by adding a detergent or mixture of detergents comprising a cationic detergent to the concentrated liquid harvest obtained in step c), wherein the final concentration of said cationic detergent is at least 2000 µg/ml,
e) separating viral cores from the composition obtained after step d),
f) optionally removing cationic detergent from the composition obtained after step e),
g) purifying haemagglutinin and/or neuraminidase by applying chromatography,
wherein step c) is followed directly by step d),
and
wherein no purification step that is directed at specifically purifying orthomyxovirus is carried out prior to step d).

2. The method according to claim 1, wherein the only chromatography step that is carried out is carried out in step g).

3. The method according to any of the preceding claims, wherein an inactivation step and/or viral clearance step is carried out.

4. The method according to any of the preceding claims, wherein the volume in step c) is reduced by a factor of at least 5.

5. The method according to any of the preceding claims, wherein the cationic detergent is based on quaternary ammonium cations.

6. The method according to any of the preceding claims, wherein step b) is directly followed by step c) which is directly followed by step d).

7. The method according to any of the preceding claims, wherein no detergent exchange takes place.

8. The method according to any of the preceding claims, wherein no nuclease or other DNA digesting enzyme, is added prior to step d), preferably no nuclease is added at any stage of the process.

9. The method according to any of the preceding claims, wherein the orthomyxovirus is influenza virus A, B or C.

10. The method according to any of the preceding claims, wherein the fluid in step a) is produced by using a disposable bioreactor.

11. The method according to any of the preceding claims, defined by features selected from any one of the following:
- step c) is carried out by applying a method selected from the group consisting of tangential flow filtration, ultracentrifugation and precipitation;
- step e) is carried out by applying a method selected from the group consisting of ultracentrifugation and tangential flow filtration; and /or
- the chromatography of step g) is selected from the group consisting of ion exchange chromatography, hydrophobic interaction chromatography, affinity chromatography and size exclusion chromatography.

12. The method according to any of the preceding claims, comprising an additional step of formulating the obtained viral antigen into a pharmaceutical composition, preferably the pharmaceutical composition is a vaccine preparation or an intermediate thereof, more preferably the pharmaceutical composition is an influenza vaccine.

13. The method according to any of the preceding claims, wherein step g) comprises the use of a composition having a conductivity in the range of between equal to or more than 3.0 to less than 5.0 mS/cm, preferably between equal to or more than 3.5 to equal to or less than 4.7 mS/cm, even more preferably in a range of between equal to or more than 3.6 and equal to or less than 4.65 mS/cm, when referenced to 25°C, and/or wherein step g) comprises the use of a composition having a molar concentration in the range of between equal to or more than 1.0 M to equal to or less than 3.5 M, preferably in the range of between equal to or more than 1.0 M to equal to or less than 3.0 M, more preferably in a range of between equal to or more than 1.5 M to equal to or less than 3.0 M, and even more preferably in a range of between equal to or more than 1.5 M to equal to or less than 2.6 M, when referenced to 25°C.

14. Method for the manufacture of a vaccine preparation containing haemagglutinin and/or neuraminidase derived from a cell-based orthomyxovirus propagation culture, comprising steps a) to g) of any of the preceding claims, and a step of formulating the obtained, purified haemagglutinin and/or neuraminidase into a vaccine preparation.

## Patentansprüche

1. Verfahren zur Herstellung von Hämagglutinin und/oder Neuraminidase abgeleitet von Orthomyxovirus, umfassend die folgenden Schritte in der angegebenen Reihenfolge:
a) Zur-Verfügung-Stellen einer Flüssigkeit abgeleitet von einer Zell-basierten Orthomyxovirus-Vermehrungskultur, oder einer Ei-basierten Orthomyxovirus-Vermehrungskultur, die dieses Virus umfasst,
b) Klären der Flüssigkeit umfassend Orthomyxovirus durch Anwenden von Zentrifugation, Tangentialfluss-Filtration oder Tiefenfiltration, resultierend in einer flüssigen Ernte umfassend dieses Virus,
c) Reduzieren des Volumens der nach Schritt b) erhaltenen flüssigen Ernte um einen Faktor von mindestens 4, um eine konzentrierte flüssige Ernte zu erhalten, wobei dieser Volumen-Reduktionsschritt in einem einzigen Schritt durchgeführt wird,
d) Solubilisieren des Orthomyxovirus, welches in der flüssigen Ernte vorhanden ist, durch Hinzufügen eines Detergens oder einer Mischung von Detergenzien umfassend ein kationisches Detergens, zu der in Schritt c) erhaltenen konzentrierten flüssigen Ernte, wobei die Endkonzentration dieses kationischen Detergens mindestens 2000 µg/ml ist,
e) Separieren der Viruskerne von der nach Schritt d) erhaltenen Zusammensetzung,
f) optional, Entfernen des kationischen Detergens von der nach Schritt e) erhaltenen Zusammensetzung,
g) Aufreinigen von Hämagglutinin und/oder Neuraminidase durch Anwenden von Chromatographie, wobei Schritt c) direkt von Schritt d) gefolgt wird, und
wobei kein Aufreiningungsschritt, der darauf abzielt, spezifisch Orthomyxovirus aufzureinigen, vor Schritt d) ausgeführt wird.

2. Verfahren gemäß Anspruch 1, wobei der einzige Chromatographie-Schritt, der durchgeführt wird, in Schritt g) durchgeführt wird.

3. Verfahren gemäß irgendeinem der vorhergehenden Ansprüche, wobei ein Inaktivierungsschritt und/oder ein Virusabreicherungsschritt durchgeführt wird.

4. Verfahren gemäß irgendeinem der vorhergehenden Ansprüche, wobei das Volumen in Schritt c) um einen Faktor von mindestens 5 reduziert wird.

5. Verfahren gemäß irgendeinem der vorhergehenden Ansprüche, wobei das kationische Detergens auf quaternären Ammonium-Kationen basiert.

6. Verfahren gemäß irgendeinem der vorhergehenden Ansprüche, wobei Schritt b) direkt von Schritt c) gefolgt wird, welcher direkt von Schritt d) gefolgt wird.

7. Verfahren gemäß irgendeinem der vorhergehenden Ansprüche, wobei kein Detergens-Austausch stattfindet.

8. Verfahren gemäß irgendeinem der vorhergehenden Ansprüche, wobei vor Schritt d) keine Nuklease oder anderes DNA-Verdauungsenzym hinzugefügt wird, bevorzugt wird keine Nuklease während irgendeines Stadiums des Verfahrens hinzugefügt.

9. Verfahren gemäß irgendeinem der vorhergehenden Ansprüche, wobei das Orthomyxovirus Influenzavirus A, B oder C ist.

10. Verfahren gemäß irgendeinem der vorhergehenden Ansprüche, wobei die Flüssigkeit in Schritt a) unter Verwendung eines Einwegbioreaktors hergestellt wird.

11. Verfahren gemäß irgendeinem der vorhergehenden Ansprüche, definiert durch Eigenschaften ausgewählt aus irgendeiner der folgenden:
- Schritt c) wird durch Anwenden eines Verfahrens ausgewählt aus der Gruppe bestehend aus Tangentialfluss-Filtration, Ultrazentrifugation und Präzipitation durchgeführt;
- Schritt e) wird durch Anwenden eines Verfahrens ausgewählt aus der Gruppe bestehend aus Ultrazentrifugation und Tangentialfluss-Filtration durchgeführt; und/oder
- die Chromatographie von Schritt g) ist ausgewählt aus der Gruppe bestehend aus Ionenaustauschchromatographie, hydrophobe Interaktionschromatographie, Affinitätschromatographie und Größenausschlusschromatographie.

12. Verfahren gemäß irgendeinem der vorhergehenden Ansprüche, umfassend einen zusätzlichen Schritt der Formulierung des erhaltenen Virus-Antigens in eine pharmazeutische Zusammensetzung, bevorzugt ist die pharmazeutische Zusammensetzung eine Vakzin-Zubereitung oder ein Zwischenprodukt davon, mehr bevorzugt ist die pharmazeutische Zusammensetzung ein Influenzavakzin.

13. Verfahren gemäß irgendeinem der vorhergehenden Ansprüche, wobei Schritt g) die Verwendung einer Zusammensetzung mit einer Leitfähigkeit in dem Bereich zwischen gleich oder mehr als 3,0 bis weniger als 5,0 mS/cm, bevorzugt zwischen gleich oder mehr als 3,5 bis gleich oder weniger als 4,7 mS/cm, noch mehr bevorzugt in einem Bereich zwischen gleich oder mehr als 3,6 und gleich oder weniger als 4,65 mS/cm, wenn referenziert auf 25°C, umfasst, und/oder wobei Schritt g) die Verwendung einer Zusammensetzung mit einer molaren Konzentration in dem Bereich von zwischen gleich oder mehr als 1,0 M bis gleich oder weniger als 3,5 M, bevorzugt in dem Bereich von zwischen gleich oder mehr als 1,0 M bis gleich oder weniger als 3,0 M, mehr bevorzugt in einem Bereich von zwischen gleich oder mehr als 1,5 M bis gleich oder weniger als 3,0 M, und noch mehr bevorzugt in einem Bereich von zwischen gleich oder mehr als 1,5 M bis gleich oder weniger als 2,6 M, wenn referenziert auf 25°C, umfasst.

14. Verfahren zur Herstellung einer Vakzin-Zubereitung enthaltend Hämagglutinin und/oder Neuraminidase abgeleitet von einer Zell-basierten Orthomyxovirus-Vermehrungskultur, umfassend Schritte a) bis g) von irgendeinem der vorhergehenden Ansprüche, und einen Schritt des Formulierens der erhaltenen, aufgereinigten Hämagglutinin und/oder Neuraminidase in eine Vakzin-Zubereitung.

## Revendications

1. Procédé de production d'hémagglutinine et/ou de neuraminidase dérivée(s) d'orthomyxovirus, comprenant les étapes suivantes dans l'ordre indiqué :
a) la fourniture d'un fluide dérivé d'une culture de propagation d'orthomyxovirus à base de cellules ou d'une culture de propagation d'orthomyxovirus à base d'oeuf comprenant ledit virus,
b) la clarification du fluide comprenant l'orthomyxovirus par application d'une centrifugation, d'une filtration à écoulement tangentiel, ou d'une filtration en profondeur, en obtenant une récolte de liquide comprenant ledit virus,
c) la réduction du volume de la récolte de liquide obtenue après l'étape b) d'un facteur d'au moins 4 pour obtenir une récolte de liquide concentrée, dans lequel cette étape de réduction de volume est effectuée en une seule étape,
d) la solubilisation de l'orthomyxovirus présent dans la récolte de liquide en ajoutant un détergent ou un mélange de détergents comprenant un détergent cationique à la récolte de liquide concentrée obtenue à l'étape c), dans lequel la concentration finale dudit détergent cationique est d'au moins 2000 µg/mL,
e) la séparation de nucléoïdes de la composition obtenue après l'étape d),
f) l'élimination éventuelle du détergent cationique de la composition obtenue après l'étape e),
g) la purification de l'hémagglutinine et/ou de la neuraminidase en appliquant une chromatographie, dans lequel l'étape c) est suivie directement de l'étape d), et
dans lequel aucune étape de purification qui est dirigée sur la purification spécifique de l'orthomyxovirus n'est effectuée avant l'étape d).

2. Procédé selon la revendication 1, dans lequel la seule étape chromatographique qui est effectuée l'est à l'étape g).

3. Procédé selon l'une quelconque des revendications précédentes, dans lequel une étape d'inactivation et/ou une étape de clairance virale est ou sont effectuées.

4. Procédé selon l'une quelconque des revendications précédentes, dans lequel le volume à l'étape c) est réduit d'un facteur d'au moins 5.

5. Procédé selon l'une quelconque des revendications précédentes, dans lequel le détergent cationique est basé sur des cations d'ammonium quaternaire.

6. Procédé selon l'une quelconque des revendications précédentes, dans lequel l'étape b) est directement suivie de l'étape c) qui est directement suivie de l'étape d).

7. Procédé selon l'une quelconque des revendications précédentes, dans lequel aucun échange de détergent n'a lieu.

8. Procédé selon l'une quelconque des revendications précédentes, dans lequel aucune nucléase ni d'autre enzyme de digestion d'ADN n'est ajoutée avant l'étape d), de préférence aucune nucléase n'est ajoutée à un stade quelconque du procédé.

9. Procédé selon l'une quelconque des revendications précédentes, dans lequel l'orthomyxovirus est le virus de la grippe A, B ou C.

10. Procédé selon l'une quelconque des revendications précédentes, dans lequel le fluide à l'étape a) est produit en utilisant un bioréacteur jetable.

11. Procédé selon l'une quelconque des revendications précédentes, défini par des caractéristiques sélectionnées parmi l'une quelconque des suivantes :
- l'étape c) est effectuée en appliquant un procédé sélectionné dans le groupe constitué d'une filtration à écoulement tangentiel, d'une ultracentrifugation et d'une précipitation ;
- l'étape e) est effectuée en appliquant un procédé sélectionné dans le groupe constitué d'une ultracentrifugation et d'une filtration à écoulement tangentiel ; et/ou
- la chromatographie de l'étape g) est sélectionnée dans le groupe constitué d'une chromatographie par échange ionique, d'une chromatographie par interaction hydrophobe, d'une chromatographie par affinité et d'une chromatographie par exclusion de tailles.

12. Procédé selon l'une quelconque des revendications précédentes, comprenant une étape supplémentaire de formulation de l'antigène viral obtenu dans une composition pharmaceutique, de préférence la composition pharmaceutique est une préparation de vaccin ou un intermédiaire de celle-ci, mieux encore la composition pharmaceutique est un vaccin de la grippe.

13. Procédé selon l'une quelconque des revendications précédentes, dans lequel l'étape g) comprend l'utilisation d'une composition ayant une conductivité dans la plage égale ou supérieure à 3,0 à inférieure à 5,0 mS/cm, de préférence égale ou supérieure à 3,5 à égale ou inférieure à 4,7 mS/cm, mieux encore dans une plage égale ou supérieure à 3,6 à égale ou inférieure à 4,65 mS/cm, lorsque l'on se réfère à une température de 25 °C, et/ou dans lequel l'étape g) comprend l'utilisation d'une composition ayant une concentration molaire dans la plage égale ou supérieure à 1,0 M à égale ou inférieure à 3,5 M, de préférence dans la plage égale ou supérieure à 1,0 M à égale ou inférieure à 3,0 M, mieux encore dans une plage égale ou supérieure à 1,5 M à égale ou inférieure à 3,0 M et bien mieux encore dans une plage égale ou supérieure à 1,5 M à égale ou inférieure à 2,6 M, lorsqu'on se réfère à une température de 25 °C.

14. Procédé de fabrication d'une préparation de vaccin contenant de l'hémagglutinine et/ou de la neuraminidase tirée(s) d'une culture de propagation d'orthomyxovirus à base de cellules, comprenant les étapes a) à g) de l'une quelconque des revendications précédentes, et une étape de formulation de l'hémagglutinine et/ou de la neuraminidase purifiée(s) obtenue(s) dans une préparation de vaccin.
